# EUROPEAN PATENT APPLICATION

(11) **EP 1 844 713 A1**
(43) Date of publication of application: **17.10.2007**
(21) Application number: 07104167.7
(22) Date of filing: 14.03.2007
(51) Int. Cl.: A61B 17/00

(54) **Implantable closure device**

(30) Priority: 22.03.2006 US 386081
(71) Applicant: RADI MEDICAL SYSTEMS AB, 754 50 Uppsala (SE)
(72) Inventor: Preinitz, Fredrik, 753 50, UPPSALA (SE); Fällman, David, 753 29, UPPSALA (SE); Egnelöv, Per;c/o Radi Med. Systems Co,.Ltd, Paklok Sub-District,Thalang District, Phuket 83110 (TH)
(74) Representative: Holmberg, Nils Anders Patrik

(57) **Abstract**

A medical closure device (20) is provided, comprising a tubular member (21) provided with first struts (22) that extend between a first end portion (24) and a central portion (25) and second struts (23) that extend between said central portion and a second end portion (26), each strut being provided with a hinge section (27, 28), such that said closure device is movable between a first elongated introduction configuration and a second positioning configuration in which the first and/or second end portions have been moved towards each other such that the first and/or second struts have moved radially away from a longitudinal central axis of the closure device. The closure device further has a third configuration, in which a locking member (30) is positioned in the tubular member such that the first end portion abuts the radial protrusions of a first end (32) of the locking member and the radial protrusions of a second end (36) of the locking member prevents further compression of the tubular member, Additionally, the closure device has a fourth configuration in which the second end portion has been forced over the radial protrusions of a second end such that the closure device is held in an expanded and locked configuration.

## Description

### Field of the invention

The present invention relates generally to a medical device for closing an opening or defect in an organ within a living body, e.g. a septal defect in a heart or a percutaneous puncture in a vessel wall (such as walls in arteries or other blood vessels), and in particular to an expandable and repositionable closure device, which can be remotely maneuvered from an initial positioning configuration to a final configuration in which the opening or defect is closed.

### Background of the invention

The closing of an opening in an organ of a patient is a medical procedure that frequently has to be practiced by doctors or other trained medical personnel. The opening may be a hole created by the doctor for a specific and usually temporary purpose, or the opening can be a congenital or acquired defect. An example of the former would be a puncture hole created in a patient's femoral artery to obtain access to the coronary system, while an example of the latter is a septal defect in a patient's heart. For descriptive and illustrative purposes the present invention will be described with reference to such a septal defect, although such techniques can be applied to other fields of application.

As is well-known, the human heart is divided into four chambers: the left atrium, the right atrium, the left ventricle, and the right ventricle. The atria are separated from each other by the interatrial septum, and the ventricles are separated by the interventricular septum.

Either congenitally or by acquisition, abnormal openings or holes can form between the chambers of the heart, causing shunting of blood through the opening or hole. For example, with an atrial septal defect, blood is shunted from the left atrium to the right atrium, which produces an over-load of the right side of the heart. In addition to left-to-right shunts such as occur in patent ductus arteriosus from the aorta to the pulmonary artery, the left side of the heart has to work harder because some of the blood will recirculate through the lungs instead of going to the rest of the body. The ill effects of such lesions usually cause added strain to the heart with ultimate failure if not corrected.

One way to cure a septal defect in the septum of a heart is to position and anchor a specially designed closure device at the septum such that both sides of the septal defect are spanned by the closure device to thereby close the defect. Examples of such septal defect closure devices are known from the U.S. Patent Nos. 5,853,422; 6,024,756; 6,117,159 and 6,312,446 to Huebsch et al., which disclose a closure device comprising a cylindrical shaft of metal or polymeric material with concentric parallel cuts through the wall of the device to thereby create flattened support struts. The centers of the support struts are intended to move radially away from the longitudinal axis of the device in a hinge-like fashion in response to movements of the proximal and distal ends of the device towards the center thereof. The closure device is claimed to be reversibly operable between a delivery configuration and a defect closing configuration, in which the closure device can be locked. No means are, however, provided to create a well-defined, user-perceivable transition between the closing configuration and the locked configuration.

A similar septal defect closure device is also disclosed in European patent application EP1651116 to Chanduszko.

Within the medical field it is of utmost importance that closure devices work properly, and the general object of the present invention is therefore to improve a closure device of the aforementioned type in such a way that a safe and user-friendly medical device is obtained, whose movements and configurations can be sensed and controlled in a more reliable way in comparison with the previously known devices.

### Summary of the invention

The above-mentioned object is achieved by the present invention according to the independent claim. Preferred embodiments are set forth in the dependent claims.

According to the present invention, a septal defect closure device comprises an elongated tubular member in which a first set of longitudinal slits or cuts have been made on a first side of an uncut central portion and a second set of longitudinal slits or cuts have been made on the opposite side of the central portion. On each side of the central portion, the slits extend towards the ends of the tubular member to terminate a short distance before the respective end, such that uncut proximal and distal end portions are formed. The tubular member, which is made from a flexible and preferably resorbable material, has thereby been provided with proximal and distal sets of struts or ribs. The distal ends of the distal struts are flexibly connected to the distal end portion of the tubular member, while the proximal ends of the distal struts are flexibly connected to the central portion. Similarly, the proximal ends of the proximal struts are flexibly connected to the proximal end portion of the tubular member, while the distal ends of the proximal struts are flexibly connected to the central portion. The struts are further each provided with a hinge section such that each strut in effect is divided into two articulated arms.

When the septal defect closure device during use is compressed such that the distal and proximal end portions are forced towards each other, the hinge sections of the struts move radially out from the longitudinal central axis of the closure device, and the respective arms of the struts assume an essentially perpendicular angle to the central axis of the closure device. The septal defect closure device further comprises a central elongated locking member, which can be either separate from or already integrated with the tubular member. In the former case, a elongated locking member is inserted into the tubular member such that the distal end portion of the tubular member abuts one or several radially protruding portions of a distal end of the locking member, and the proximal end portion of the tubular member is then pushed over one or several radially protruding portions of a proximal end of the locking member. The radially protruding portions can comprise a continuous rim, a discontinuous rim or discrete radial protrusions of various dimensions. In the compressed state, the central, proximal and distal portions of the tubular member fit snugly along the central locking member, and the closure device is held in the compressed state by the enlarged distal and proximal rims or other radially protruding portions of the locking member, which prevents the closure device from resuming its original elongated shape. The device further comprises a keying feature, which prevents rotational movement of the locking member in relation to the tubular member.

In accordance with the present invention, the closure device has thereby been provided with four (4) well-defined configurations: an introduction configuration, a positioning configuration, a closed configuration, and a locked configuration, which are all well-defined and, more importantly, are easily discernible by a doctor who is implanting the closure device in, for example, the septum of a patient's heart. The usefulness of having well-defined configurations will be apparent from the detailed description below taken in conjunction with the appended drawings.

### Brief Description of the Drawings

Fig. 1 is a schematic illustration of a human heart having an atrial as well as a ventricular septal defect.
Fig. 2 is a schematic illustration of a human heart having a septal defect, which is to be closed by means of a medical procedure that, in a first step, involves the positioning of a septal defect closure device according to the present invention.
Fig. 3 illustrates an intermediate step in the medical procedure, in which a distal portion of the closure device of Fig. 2 is expanded in order to locate the septal defect from the distal side of the septal defect.
Fig. 4 illustrates another intermediate step in the medical procedure, in which a proximal portion of the closure device of Fig. 2 is expanded in order to locate the septal defect from the proximal side of the septal defect.
Fig. 5 illustrates the closure device of Fig. 2, which has been positioned in the septum to close the septal defect therein.
Fig. 6 shows a septal defect closure device according to the invention in an introduction configuration before any longitudinal compression of the closure device.
Fig. 7 shows the closure device of Fig. 6 in an intermediate semi-compressed positioning configuration.
Fig. 8 shows the closure device of Fig. 6 in another intermediate semi-compressed positioning configuration.
Fig. 9 shows a locking member which constitutes a separate part of a septal defect closure device.
Fig. 10 shows the closure device of Fig. 6 in a closed configuration.
Fig. 11 shows the closure device of Fig. 6 in another closed configuration, in which distal portions of a mechanical actuator are visible.
Fig. 12 illustrates the closure device of Fig. 6 in a final locked configuration.

### Detailed Description of Preferred Embodiments

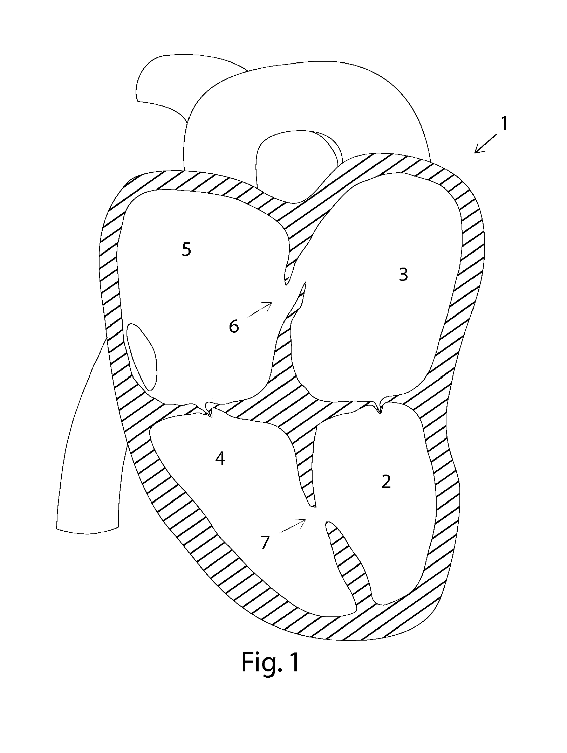

A schematic cross-sectional view of a human heart 1 is shown in Fig. 1. The heart 1, with its left ventricle 2, left atrium 3, right ventricle 4, and right atrium 5, suffers from an atrial septal defect 6 as well as a ventricular septal defect 7. Below a medical procedure will be discussed in which an atrial septal defect is closed. It should, however, be clear that a septal defect closure device according to the present invention equally well could be employed to close a ventricular septal defect such as ventricular septal defect 7 of Fig. 1. It should further be noticed that the septal defects 6, 7 can be accessed from different vessels, e.g. from the superior or inferior vena cava, or from the aorta. This implies, in turn, that throughout the present description terms like "distal" and "proximal" should always be seen from the end of a delivering catheter, through which a septal defect closure device is delivered (and not from any particular chamber or vessel of a heart).

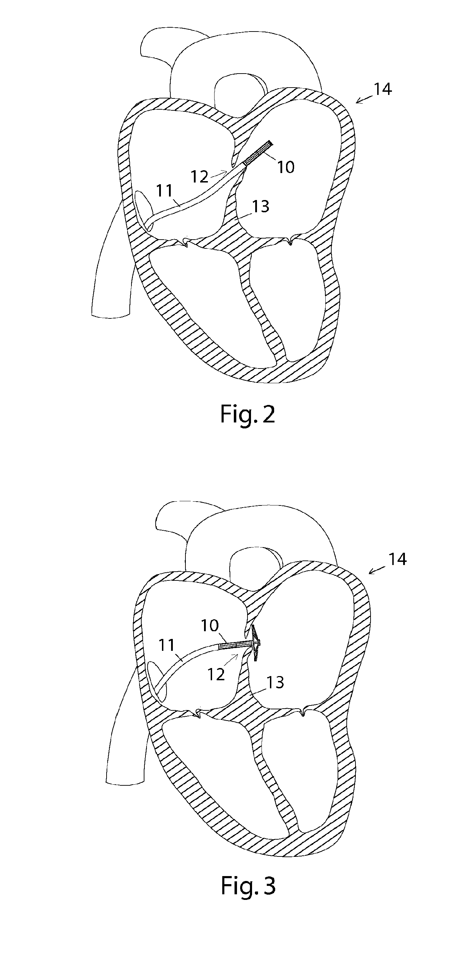

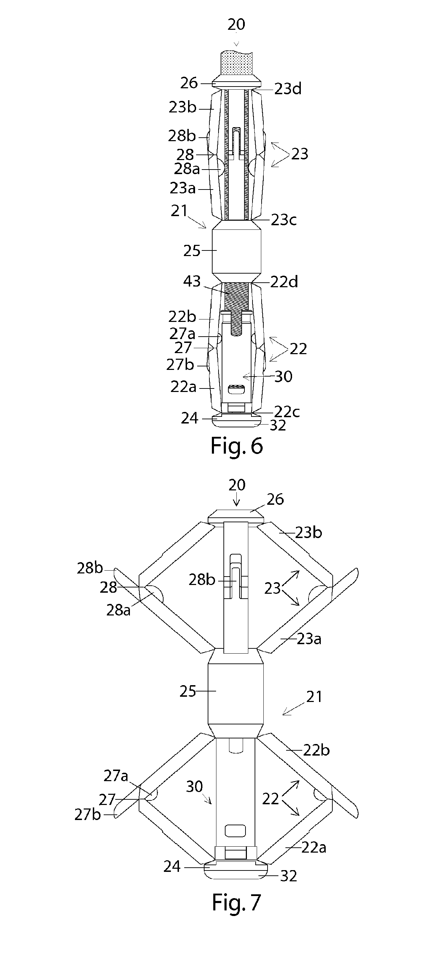

In conjunction with Figs. 2 to 5, a medical procedure will be briefly described, in which a septal defect closure device according to one embodiment of the present invention is employed to close a septal defect in the septum of a heart; and thereafter different configurations and parts of the closure device itself will be described in detail in conjunction with Figs. 6 to 12.

Fig. 2 illustrates a septal defect closure device 10 according to the present invention, which has been introduced into an atrial septal defect 12 in the atrial septum 13 of a heart 14 using a delivering catheter 11. The closure device 10 is of the same general construction that has been generally described above, and comprises an elongated tubular member in which distal and proximal sets of struts have been provided. The distal struts extend from a central portion of the closure device 10 to a distal end portion thereof, and the proximal struts extend from a proximal end portion of the closure device 10 to the central portion. As already discussed, each strut is provided with a hinge region, and each strut is thereby effectively divided into two hinge-connected arms. The characterizing details of the hinge region will be discussed further below. In Fig. 2, the closure device 10 is shown in an initial introduction configuration, in which the arms of each strut are substantially aligned with each other. In this introduction configuration, the closure device 10 therefore has a generally elongated tubular shape, which facilitates the introduction of the closure device 10 into the artery and heart of a patient. The introduction configuration is defined as the configuration that the closure device assumes by itself, i.e. without any compression being induced by a mechanical actuator (not shown in the figure) connected to the closure device. In this introduction configuration, the closure device has therefore a generally tubular shape, although the closure device could be preformed such that the arms of each strut exhibit a small positive angle in relation to each other. Such a positive angle guarantees the proper radial expansion of the tubular member during longitudinal compression of the tubular member.

To ascertain correct positioning of the closure device 10 with respect to the septal defect 12, the distal set of struts can be moved radially outwards from the central axis of the closure device 10, such that a partly expanded configuration is obtained. The radial movements of the distal struts are effectuated by partially compressing the closure device 10 through the maneuvering of a mechanical actuator (not shown in Figs. 2-5). In this semi-expanded locating or positioning configuration, the closure device 10 is retracted until the distal struts abut the distal side of the atrial septum 13 surrounding the septal defect 12. The septal defect 12 can thereby be located by a doctor, who in this phase of the medical procedure will feel a marked increase in resistance against further retraction. This intermediate step of the medical procedure is depicted in Fig. 3.

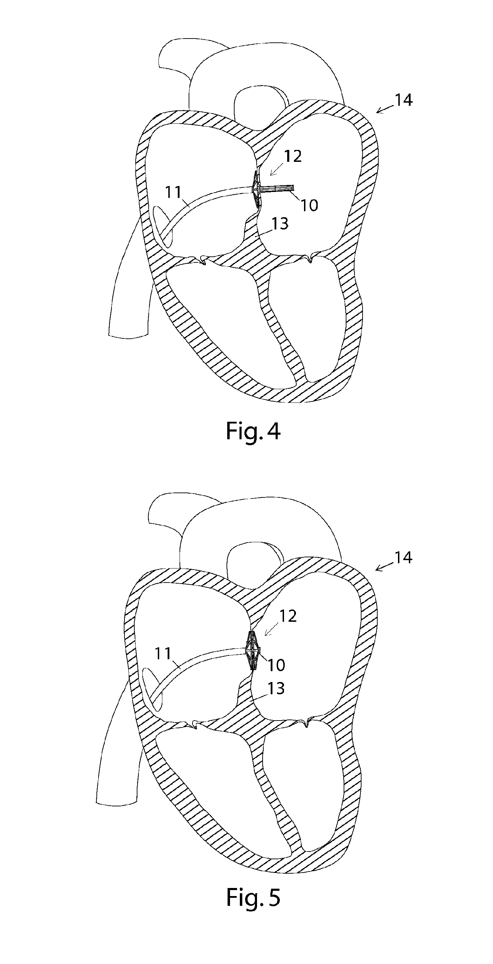

As an alternative or complement, the proximal set of struts can be moved radially outwards from the central axis of the closure device 10, such that another partly expanded positioning configuration is obtained. As before, the radial movements of the proximal struts are accomplished by partially compressing the closure device 10 through the maneuvering of the mechanical actuator mentioned above. In this second semi-expanded locating or positioning configuration, the closure device 10 is advanced until the proximal struts abut the proximal side of the atrial septum 13 surrounding the septal defect 12. The septal defect 12 can thereby be located by a doctor who in this phase of the medical procedure will feel a marked increase in resistance against further advancement. This intermediate step of the medical procedure is depicted in Fig. 4. It should be mentioned that the closure device 10 can be reversibly moved between the elongated tubular introduction configuration of Fig. 2 and either of the intermediate positioning configurations shown in Fig. 3 and Fig. 4, respectively. The closure device 10 can also assume a further intermediate positioning or locating configuration in which the proximal struts as well as the distal struts have been moved radially outwards from the central axis of the closure device 10, as will be further discussed below.

When the atrial septum 13 and thereby the septal defect 12 have been correctly located, either by the step shown in Fig. 3 or by the step of Fig. 4, or by a combination of both steps, the closure device 10 is fully expanded such that the proximal struts as well as the distal struts are forced radially outwards by maneuvering of the mechanical actuator mentioned above. In this septal defect closing configuration, the closure device 10 spans both the distal side and the proximal side of the septal defect 12, and is then held in this position. As can be seen in Fig. 5, the closure device 10 sandwiches the atrial septum 13 to thereby close the septal defect 12 therein. It should be mentioned that the term "close" or similar terms used herein in conjunction with the description of the closing of a septal defect should not be taken too literally. Such terms are meant to encompass all stages from actually sealing or closing off a septal defect to merely restricting the flow of blood therethrough, the important feature being that the closure device permits and facilitates healing of the septal (or other type of) defect over time. To improve the sealing capability of a closure device of the present type, it is conceivable that the distal and/or proximal struts at least partly are covered by a thin membrane or formed integrally with a thin membrane, which preferably is made from a resorbable material.

A special feature of the closed configuration illustrated in Fig. 5 is that the closure device 10 still is repositionable. This means that by using a mechanical actuator, the closure device 10 is reversibly movable between the configurations described above in conjunction with Figs. 2-5, i.e. from the closed configuration of Fig. 5, to anyone of the intermediate positioning configurations of Fig. 3 or Fig. 4, and back to the original introduction configuration of Fig. 2. The closure device 10 can then be retracted out of the patient's body and be disposed, or can once again be positioned by repeating the steps illustrated above. The closed configuration of the closure device 10 is defined as the extreme end position of the different and gradually changing positioning configurations. In the closed configuration essentially no further compression of the closure device 10 is possible while still maintaining a reversibly movable closure device 10. The latter will be thoroughly discussed below.

In accordance with the present invention, a closure device encompasses a fourth configuration, in which the closure device is irreversibly locked. The transition from the closed configuration to this locked configuration is effectuated by the mechanical actuator mentioned above. A special feature of the present closure device is that a doctor will feel when the closed configuration has been reached, so that he or she can decide whether the mechanical actuator should be maneuvered such that the final locked configuration is achieved. Having in mind that the closed configuration constitutes a situation from which the closure device can be removed, whereas the locked configuration implies a non-retrievable closure device, the importance of having a well-defined transition between these two states should be appreciated. Also this feature will be further discussed below.

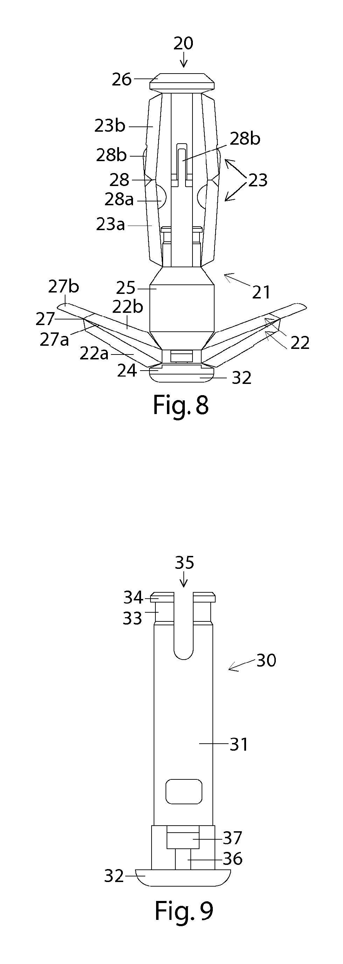

An embodiment of a septal defect closure device 20 according to the present invention is illustrated in Fig. 6. Fig. 6 shows the closure device 20 in a first or introduction configuration in which the closure device 20 has the general shape of an elongated tubular member 21, through which a number of longitudinal, parallel cuts or slits have been made to thereby form a first or distal set of struts 22 and a second or proximal set of struts 23. The first strut set 22 extends between a first end portion 24 of the tubular member 21 and a central portion 25 thereof, while the second strut set 23 extends between the central portion 25 and a second end portion 26 of the tubular member 21. The first and second end portions 24, 26 as well as the central portion 25 are uncut and are preferably shorter than the slit portions of the tubular member 21. Somewhere along the length of the first set of struts 22, the tubular member 21 has been provided with a hinge region 27, which will thereby act as a hinge or articulation 27, effectively dividing each strut 22 into two articulated arms: a first or distal arm 22a and a second or proximal arm 22b. Similarly, the struts in the second set of struts 23 are each provided with a hinge section 28, which in effect divides each strut 23 into two articulated arms: a first or distal arm 23a and a second or proximal arm 23b. In Fig. 6, the device has been provided with two distal arms 22 and four proximal arms 23; however the number of arms for each set can be varied without departing from the scope of the present invention, as will be further described below. In addition, the closure device 20 comprises further a locking member 30, which is further described below in connection with Fig. 9.

In the figures, the hinge region has been constructed so as to comprise a middle longitudinally extended section which will constitute an extra protruding arm 27b, 28b when folded, which is more apparent in Figs. 7-8 and Figs. 10-12. In addition, the hinge regions have been provided with a protrusion 27a, 28a, which serves to keep the two connected arms 22a, 22b or 23a, 23b at an angle towards each other even in the initial introduction configuration of the tubular member, as described above. This protrusion encounters either the central holder member 43 or the locking member 30, if present, in the initial configuration of the arms, which prevents the two connected arms from aligning completely with each other. It should however be noted that it is within the scope of the present invention that the central hinge regions can be constructed by simply removing material on the outer side of the struts, thus creating weakened sections that will act as hinges, lacking the protruding extra arms 27b, 28b and/or the protrusions 27a, 28a on the inside of the hinge region.

It is should further be emphasized that the term "tubular" is merely intended to indicate the general shape of an elongated, hollow member, which comprises a number of struts, the ends of which are connected to ring-shaped or essentially cylindrical members, and which in a first introduction configuration assumes an essentially tubular shape. In other words, a tubular member, like tubular member 21, does not actually have to be cut or slit in order to create distal and proximal struts. On the contrary, a tubular member, having struts with hinge regions, as well as ring-shaped central, distal and proximal end portions, can advantageously be directly produced in this form, e.g. by injection molding or die casting. Furthermore, the struts of a tubular member, like tubular member 21, do not have to be exactly aligned with each other. Instead, a tubular member can be preformed in such a way that the two arms of a strut exhibit an angled relation to each other, to thereby guarantee that the arms actually bend outwards during compression of the tubular member. Nevertheless, the definition of the introduction configuration is still the configuration or state wherein a closure device has not been subjected to any compression by means of a mechanical actuator. The introduction configuration may therefore also be regarded as the "natural" state of the closure device.

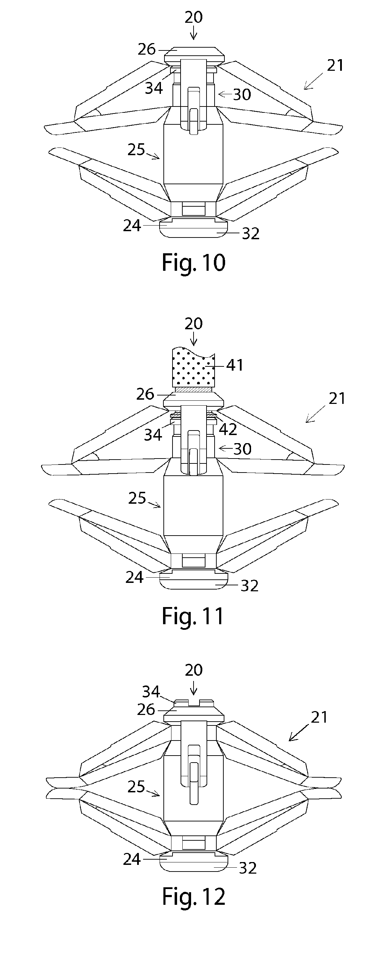

In Fig. 7, the closure device 20 of Fig. 6 is depicted in a semi-expanded positioning configuration in which the distal and proximal end portions 24, 26 of the closure device 20 have been moved towards the central portion 25. The hinge sections 27, 28 of the first and second struts 22, 23 have thereby been forced to move outwards from the central axis of the closure device 20, and the articulated arms 22a, 22b and 23a, 23b have assumed a more angled relation to the central axis of the closure device 20. Here it should be recognized that the configuration shown in Fig. 7 partly is for illustrative purposes; in practice either of the two end portions 24, 26 could be moved towards the central portion 25, to assume the locating configurations shown in Fig. 3 and Fig. 4, respectively. Another example of a positioning configuration is illustrated in Fig. 8. The semi-expanded configuration of Fig. 7 could, however, also be used to determine the proper position for the closure device 20, and can also be regarded as a positioning configuration prior to a closed configuration described below in conjunction with Fig. 10 or Fig. 11. The positioning configuration is consequently defined as all intermediate states between the introduction configuration defined above and the closed configuration, which will be described and defined below.

As can be seen in Figs. 6-8, the closure device 20 comprises further a locking member 30, which is separately illustrated in Fig. 9. The locking member 30, which according to the invention can constitute either a separate or an integrated part of closure device 20, comprises a hollow tubular body 31, which along the central part of its length is provided with an outer diameter approximately equivalent to the inner diameter of the tubular member 21 of the closure device 20. More specifically, the locking member 30 comprises a distal end rim 32, an intermediate body 31, a proximal groove 33, and a proximal end rim 34. The distance between the distal end rim 32 and the proximal end rim 34 is considerably smaller than the length of the tubular member 21. As the observant reader already may have appreciated, the diameter of the distal end rim 32 is larger than the inner diameter of the distal end portion 24 of the tubular member 21. In turn, the inner diameter of the distal end portion 24 is marginally larger than the diameter of the intermediate portion 31 of the locking member 30, such that the distal end portion 24 of the tubular member 21 can slide over the locking member 30 until the distal end portion 24 abuts the distal end rim 32 In this position, as seen in e.g. Figs. 6-8, locking knob 37 functions by locking the distal end portion 24 against the distal end rim 32, such that the tubular member and the locking member are locked at their distal ends. In addition, to prevent rotational movement of the tubular member 21 relative to the locking member 30, a keying feature between the locking member and the tubular member is provided. In one embodiment, shown in part in Fig. 9, the locking member is supplied with a protrusion 36 which is matched by a recess on the inner surface of distal end portion 24 (not shown in the figures).

Similarly to the inner diameter of distal end portion 24, the inner diameters of the central portion 25 and the proximal end portion 26 of the tubular member 21 are marginally larger than the outer diameter of the intermediate portion 31 of the locking member 30. Additionally, the outer diameter of the proximal end rim 34 is slightly larger than the inner diameter of the proximal end portion 26. During use, the proximal end portion 26 of the tubular member 21, which is made from a somewhat elastic material, must therefore be forced over the proximal end rim 34 and can then slide on the intermediate portion 31. As can be seen in Fig. 9, the locking member 30 preferably comprises a recess 35, which in combination with the groove 33 provide the proximal end rim 34 with certain resilience, facilitating the sliding of the proximal end portion 26 of the closure device 20 over the proximal end rim 34 of the locking member 30.

In the embodiment of the locking member shown in Fig. 9 the distal and proximal ends 32, 34 comprise a completely circumferential circular rim and a discontinuous circumferential rim, respectively. It should be noted that the locking member's distal and proximal ends can comprise other shapes, provided that the distal end 32 supplies a point of hindrance for the distal end portion 24 and that the proximal end 34 both allows proximal end portion 26 to be forced over it and locks the final expanded state of the device in place. Many shapes are conceivable such that at least one outer cross-sectional dimension of the distal end 32 is larger than at least one inner cross-sectional dimension of the end portion 24 and at least one outer cross-sectional dimension of the proximal end 34 is adapted to, i.e. is slightly larger than, at least one inner cross-sectional dimension of the end portion 26. One such possibility is to supply the ends with one or several radial protrusions.

As indicated above, the closure device 20 can assume an infinite number of positioning configurations during a positioning operation in which a septal defect is located and the closure device 20 is positioned therein. According to the present invention, there is, however, a well-defined endpoint for the positioning operation. This endpoint, which is referred to as the closed configuration of the closure device 20, is illustrated in Fig. 10, where it can be seen that the central portion 25 of the tubular member 21 has been positioned over the intermediate portion 31 of the locking member 30, while the proximal end portion 26 of the tubular member 21 abuts the proximal end rim 34 of the locking member 30. As has been mentioned above, the inner diameter of the proximal end portion 26 is slightly less than the diameter of the proximal end rim 34, which implies that further compression of the tubular member 21 is not possible - unless extra force is applied such that the proximal end portion 26 is forced over the proximal end rim 34. The closed configuration of Fig. 10 thereby constitutes a well-defined state.

However, the definition above, that a closed configuration is a configuration in which no further compression of the tubular member 21 is possible without forcing proximal end portion 26 over proximal end rim 34 allows for additional examples of a closed configuration. In practice, the movements of the closure device are effectuated by the previously mentioned mechanical actuator, parts of an example of which are illustrated in Fig. 11 together with the tubular member 21 as well as the locking member 30. The mechanical actuator comprises a pusher tube 41 and an actuating member 42. By moving the actuating member 42 back and forth, a doctor can during a preceding positioning operation let the tubular member 21 assume different positioning configurations, to thereby locate a septal defect (or some other type of tissue opening, e.g. a percutaneous puncture in an artery wall) and position the closure device 20 in the opening of the defect. The movements of the tubular member 21 is actually accomplished in co-operation with a hold and release member (not shown in the figure), which holds the locking member 30 in a reversible manner. The compression of the tubular member is achieved by the relative motion between the hold and release member and the actuating member 42. In the situation illustrated in Fig. 11, the distal end of the actuating member 42 abuts the proximal end rim 34 of the locking member 30. Thus, Fig. 11 illustrates a well-defined end position for the positioning operation, in which no further compression of the tubular member 21 is possible by maneuvering of the actuating member 42 in relation to the hold and release member without forcing proximal end portion 26 over proximal end rim 34. If, on the other hand, an actuating member were engaged inside a proximal end portion of a tubular member, the situation would resemble the situation illustrated in Fig. 10, i.e. a well-defined end point of the positioning operation - in which no further compression of the tubular member is possible without forcing an end portion over an end rim - would be when a proximal end portion of the tubular member abuts a proximal end rim of a locking member. The closed configuration of a closure device according to the present invention is thereby defined as the extreme end position of the positioning configurations, wherein an end portion of a locking member prevents further compression of a tubular member. This definition also encompasses closure devices where a proximal end portion of a locking member prevents further compression of a tubular member, i.e. a closure device where a distal end portion of a tubular member is pulled over an enlarged distal end rim of a locking member rather than - as in the closure device described above - having a proximal end portion of a tubular member that is pushed over an enlarged proximal end rim of a locking member. It should be kept in mind that, as mentioned above, the locking member's distal and proximal ends can also comprise other shapes, such as any variation of radial protrusion, as long as the cross-sectional dimensions of the end portions allow the progression through the four different configurations as described herein.

From Fig. 11 it may be realized that when the actuating member 42 abuts the proximal end rim 34 of the locking member 30, the closure device 20 can be transferred into the final locked state by movement of the pusher tube 41. To accomplish this, the pusher tube 41 (which can slide with respect to actuating member 42) is advanced, so that the proximal end portion 26 of the tubular member 21 is forced up and over the proximal end rim 34 of the locking member 30. This movement requires that the proximal end portion 26 and/or the proximal end rim 34 possesses a certain degree of resilience.

The final locked configuration of the closure device 20 is illustrated in Fig. 12, in which the distal and proximal end portions 24, 26 of the tubular member 21 have been fully moved towards each other until the central portion 25 is positioned over the tubular body 31 of the locking member 30 and the proximal end portion 26 has been moved over the proximal end rim 34 of the locking member 30. The closure device 20 is held in this compressed state due to the enlarged distal and proximal end rims 32, 34 of the locking member 30, which have diameters larger than the distal end portion 24 and the proximal end portion 26, respectively. The closure device 20 can then be released and left in this locked configuration by maneuvering of the hold and release member mentioned above. The locked configuration of a closure device is thereby defined as the configuration in which the closure device is fully expanded, and in which the closure device can be held without assistance of a mechanical actuator.

It should be noted that it is also within the scope of the invention to vary specific dimensions of the closure device, which provides the advantage of being able to adapt a closure device to both the particular anatomy of the underlying tissue and the local physiology (e.g. the flow or coagulation tendency of the blood). It may, for example, be desirable to arrange a closure device in such a way that the left part of the closure device, i.e. the part that is implanted into the left atrium of a heart, is smaller than the right part of the closure device, to thereby reduce the amount of artificial material introduced into the left atrium, which in turn may reduce the formation of thrombogenic material therein. One example of varying the device's dimensions is to provide a closure device having proximal struts with one length and distal struts with a different length. Another is to vary the length and/or outer diameter of a central portion of a tubular member, thereby creating the possibility to adapt the device to a particular anatomy of the underlying tissue. Similarly, it is also possible to have different lengths of the articulated arms within a strut set, such that, for example, the distal arms are longer than the proximal arms, or vice versa. Furthermore, it is also within the scope of the invention to use any number of struts per set, preferably between 1 and 10 struts per set, more preferably between 2 and 6 struts per set. In addition, it can be advantageous to provide a closure device in the form of two separate tubular members (and a separate locking member) as this would provide a doctor with the possibility to tailor a septal defect closure device to the specific medical situation at hand, without the necessity of producing an excessive large number of closure devices with different dimensions.

In this context, it should be recognized that it is not mandatory that a heart is accessed via the venous system, as is shown in Figs. 2 to 5, but the heart could be accessed via the arterial side. This implies that if a doctor wishes to place a smaller part of a closure device at the left side of a heart than at the right side of the heart, then this smaller part (e.g. the shorter struts) will constitute the distal set of struts if the heart is accessed via the venous system, whereas the smaller part will constitute the proximal set of struts if the heart is accessed through the arterial system.

It has already been mentioned that a locking member can constitute a separate part of a closure device, and a locking member can be made from a first material and a tubular member can be made from a second material. With different materials some specific advantages can be achieved. If, for example, the closure device is a resorbable closure device, then the resorption time of the material in the locking member can be different from the resorption time of the material in the tubular member, such that the mechanical properties of the closure device are maintained until the surrounding tissue has healed to the point where the support of the closure device is not necessary anymore. Further, whether or not the materials are resorbable materials, different requirements are put on the different pieces. For example, the material in the hinge portions of a tubular member must be ductile and have a high durability, whereas the locking member must have a rather high stiffness. Furthermore, it may be necessary to have one material in a locking member and another material in a tubular member in order to match the resorption times due to different dimensions of the members involved in a resorbable closure device.

Examples of resorbable materials for the tubular member and the locking member may include, but are not limited to, those materials made from aliphatic polyesters, polyether esters, and polycarbonates. More specifically, synthetic resorbable polymers such as homopolymers and copolymers made from any of the monomers lactide, glycolide, epsilon-caprolactone, trimethylene carbonate, and paradioxanone are advantageous because of their long clinical use. Other lactones that may be used together with any of the abovementioned monomers to make copolymers of various properties are valerolactone, b-butyrolactone and dioxepanone, however also other 4, 5, 6 and 7 member lactones may be of interest to obtain the characteristic material properties needed to fulfill a smooth operation of the invented closure device.

The tubular member could preferably be made from a semi-crystalline material with a lower modulus than the locking member. As previously stated, the device could, e.g. because of the hinge portions, have a more flexible material in the tubular member. Such material is preferably made from a block copolymer characterized by having a soft middle part distinguished by having a glass transition temperature below body temperature and a semi-crystalline part at each end of the soft middle part. The semi-crystalline part could be polymerized from any of the monomers glycolide, lactide, or paradioxanone. Since polyparadioxanone is a relatively soft and pliable material compared to polyglycolide and polylactide, the tubular member can be made from pure polyparadioxanone itself.

The locking member can be made from any of the above materials, but to secure the locking mechanism it is advantageous if the material is stiffer than the material used in the tubular member. The material should also preferably resorb at a somewhat slower pace than the tubular member. The locking member could also be made from amorphous or semi-crystalline material, and preferably from homopolymers or copolymers where the main monomer component is lactide, caprolactone, or paradioxanone.

Further examples of synthetic resorbable polymers that may be used in the tubular or locking member of the invented closure device are resorbable polymers made from dicarboxylic acids such as succinic, glutaric, adipic or pimelic acids with various forms of diols, polymers composed of segmented blocks of polyethyleneglycol and butyleneterephthalate, various forms of tyrosine carbonate polymers, phosphazene polymers, orthoester polymers or resorbable polyurethanes.

A particular advantage of the groups of synthetic resorbable polymers mentioned above is that various mechanical properties can be accomplished by simply changing the monomer composition in the homopolymer or copolymer. Further, in contrast to natural biopolymers, these materials can be molded and machined into complex structures, and by varying the monomer composition large time spans can be achieved for their resorption times.

It may be appreciated that it can be advantageous to provide a radiopaque closure device which is visible in an X-ray machine. When the closure device is made from a synthetic resorbable polymer, a radiopaque closure device can conveniently be produced by mixing the polymer with a suitable radiopaque agent. A suitable radiopaque agent is barium sulfate, which can be blended into the polymer or copolymer in an amount between 5% and 50%, and more preferably in an amount of 15% to 30%, to obtain the opacity needed in order to locate the closure device during an X-ray observation. Radiopaque materials can be used in a tubular member of the closure device, but is preferably used in the locking member, which marks the center of the device. The radiopaque agent, e.g. barium sulfate, can be - instead of being mixed with the polymer - introduced into preformed holes in the closure device, which are then sealed by a synthetic resorbable material. As an alternative, preformed holes can be plugged with a resorbable material containing a large amount of a radiopaque agent, e.g. barium sulfate.

Although the present invention has been described with reference to specific embodiments, also shown in the appended drawings, it will be apparent for those skilled in the art that many variations and modifications can be done within the scope of the invention as described in the specification and defined with reference to the claims below. The arms, struts and hinge regions need not have the shape shown in the drawings. As mentioned above, it should in particular be noted that the lengths of the struts can be varied, such that, for example, the length of the proximal struts is longer than the length of the distal struts, or vice versa. As mentioned, it is possible to have different lengths of the articulated arms within a strut set, such that, for example, the distal arms are longer than the proximal arms, or vice versa. The shape and design of the hinges can be varied within the scope of the invention. The hinge action could, for example, be accomplished by real hinges arranged along the struts.

## Claims

1. Medical closure device having a longitudinal central axis comprising:
a tubular member (21) having a length and a first set of struts (22) extending between a first end portion (24) and a central portion (25) and a second set of struts (23) extending between said central portion and a second end portion (26), and each strut is provided with a hinge section (27, 28) that acts as a hinge, such that said closure device is movable between a first elongated introduction configuration and a second positioning configuration in which the first and second end portions have been moved relative to each other towards each other such that said hinge sections of the first and/or second sets of struts have moved radially away from said longitudinal central axis, and
the closure device further comprises a locking member (30), which has a first end (32) and a second end (34), and said first and second ends comprise radial protrusions, and said first end (32) has at least one outer cross-sectional dimension larger than at least one inner cross-sectional dimension of the first end portion (24) and said second end (34) has at least one outer cross-sectional dimension larger than at least one inner cross-sectional dimension of the second end portion (26), and the distance between the first and second ends is smaller than the length of the tubular member, **characterized in that**
the closure device has a third closed configuration in which the locking member is positioned in the tubular member such that the first end portion (24) abuts the radial protrusions of the first end (32) and at least one enlarged inner cross-sectional dimension of the second end (34) prevents further compression of the tubular member, and
the medical closure device has a fourth configuration in which the second end portion (26) has been moved over the radial protrusions of the second end (34) such that the closure device is held in an expanded and locked configuration.

2. The medical closure device according to claim 1, **characterized in that** the radial protrusions comprise a circumferential rim or a discontinuous circumferential rim.

3. The medical closure device according to claim 1, **characterized in that** at least one of the first and second sets of struts comprises between 1 and 10 struts.

4. The medical closure device according to claim 1, **characterized in that** at least one of the first and second sets of struts is at least partly covered by a membrane.

5. The medical closure device according to claim 1, **characterized in that** said tubular member comprises two separate halves such that the respective halves can move independently of each other.

6. The medical closure device according to claim 1, **characterized in that** the closure device at least partly is made from a synthetic resorbable polymer.

7. The medical closure device according to claim 1, **characterized in that** said locking member constitutes a separate part of the closure device.

8. The medical closure device according to claim 1, **characterized in that** said locking member is made from a first material and said tubular member is made from a second material.

9. The medical closure device according to claim 1, **characterized in that** the closure device comprises a radiopaque agent.

10. The medical closure device according to claim 1, **characterized in that** the closure device is adapted for closing a septal defect in a heart.

11. The medical closure device according to claim 1, **characterized in that** the closure device is adapted for closing a puncture in a vessel wall.
